# EUROPÄISCHE PATENTSCHRIFT

(11) **EP 3 558 910 B1**
(45) Veröffentlichungstag und Bekanntmachung des Hinweises auf die Patenterteilung: **02.12.2020**
(21) Anmeldenummer: 17822686.6
(22) Anmeldetag: 22.12.2017
(51) Int. Cl.: C07C 5/48, C07C 11/04

(54) **VERFAHREN UND ANLAGE ZUR HERSTELLUNG EINES OLEFINS**
METHOD AND ASSEMBLY FOR MANUFACTURING AN OLEFIN
PROCÉDÉ ET INSTALLATION DE PRODUCTION D'UNE OLÉFINE

(30) Priorität: 22.12.2016 EP 16206447
(43) Veröffentlichungstag der Anmeldung: 30.10.2019
(73) Patentinhaber: Linde GmbH, 82049 Pullach (DE)
(72) Erfinder: ZELLHUBER, Mathieu, 82152 Martinsried (DE); SCHUBERT, Martin, 81375 München (DE); WINKLER, Florian, 81241 München (DE); MEISWINKEL, Andreas, 83253 Rimsting (DE)
(74) Vertreter: m patent group
(86) Internationale Anmeldenummer: PCT/EP2017/084341
(87) Internationale Veröffentlichungsnummer: WO 2018/115418

(56) Entgegenhaltungen:
- WO-A1-2013/148006
- US-A1- 2010 256 432

## Beschreibung

Die Erfindung betrifft ein Verfahren zur Herstellung eines Olefins und eine entsprechende Anlage gemäß den Oberbegriffen der unabhängigen Patentansprüche.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen keine thermodynamische Gleichgewichtslimitierung. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine insitu-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und Lopez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen.

Bei der ODH werden insbesondere bei Verwendung von MoVNbTeOx-Katalysatoren unter industriell relevanten Reaktionsbedingungen signifikante Mengen der jeweiligen Carbonsäuren der eingesetzten Paraffine als Nebenprodukte gebildet. Für einen wirtschaftlichen Anlagenbetrieb ist eine entsprechende Koppelproduktion von Olefinen und der jeweiligen Carbonsäuren bei Verwendung des beschriebenen Katalysatortyps in der Regel erforderlich. Dies gilt insbesondere für die Herstellung von Ethylen durch ODH von Ethan (ODH-E), bei der gleichzeitig Essigsäure gebildet wird.

Die Erfindung wird nachfolgend insbesondere unter Bezugnahme auf die ODH von Ethan (sogenannte ODH-E) beschrieben. Ihr Einsatz ist jedoch grundsätzlich auch bei der ODH höherer Paraffine wie Propan und Butan möglich und vorteilhaft.

Bei Prozessen auf Grundlage oxidativer Reaktionen wie der ODH, insbesondere der ODH-E, müssen aufgrund der Exothermie eine wirkungsvolle Temperaturkontrolle und Abführung der entstehenden Reaktionswärme erfolgen. Dabei muss stets die Gefahr eines thermischen Durchgangs unterbunden werden. Ferner entstehen bei entsprechenden Reaktionen neben dem gewünschten Zielprodukt (im Falle der ODH-E Ethylen) weitere sauerstoffhaltige Spezies wie die erwähnten Carbonsäuren, aber auch beispielsweise Carbonylverbindungen. Bei zu hohen Temperaturen wird insbesondere die unerwünschte Bildung der Nebenprodukte Kohlenmonoxid und Kohlendioxid gefördert, so dass es wünschenswert ist, Temperaturen und Temperaturgradienten in geeigneten Grenzen zu halten.

Die US 2010/0256432 A1 und die WO 2013/148006 A1 geben an, dass Wasserdampf als Wärmemoderator in der ODH eingesetzt werden kann, d.h. dass sich durch eine erhöhte Wasserdampfmenge eine übermäßige Erwärmung verhindern lässt, und dass durch eine Verdünnung mit Wasserdampf ein nichtentzündlicher Reaktionseinsatz bereitgestellt werden kann.

Eine der wesentlichen Herausforderungen bei entsprechenden Prozessen ist daher die Beherrschung der exothermen Reaktion. Dies erfolgt herkömmlicherweise durch Verdünnung des Reaktionsgases und/oder eine geeignete Kühlung des Reaktionsvolumens. Die vorliegende Erfindung stellt sich die Aufgabe, die Beherrschbarkeit entsprechender Prozesse auf Grundlage der ODH, insbesondere der ODH-E, weiter zu verbessern und Nachteile herkömmlicher Verfahren zu überwinden.

### Offenbarung der Erfindung

Vor diesem Hintergrund schlägt die vorliegende Erfindung ein Verfahren zur Herstellung eines Olefins, insbesondere von Ethylen, und eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Stoffströme, Gasgemische usw. können im hier verwendeten Sprachgebrauch reich oder arm an einer oder mehreren Komponenten sein, wobei die Angabe "reich" für einen Gehalt von wenigstens 95%, 96%, 97%, 98%, 99%, 99,5%, 99,9% oder 99,99% und die Angabe "arm" für einen Gehalt von höchstens 5%, 4%, 3%, 2%, 1%, 0,5%, 0,1% oder 0,01% auf molarer, Gewichts- oder Volumenbasis stehen kann. Sind mehrere Komponenten angegeben, bezieht sich die Angabe "reich" oder "arm" auf die Summe aller Komponenten. Ist hier beispielsweise von "Sauerstoff" oder "Ethan" die Rede, kann es sich um ein Reingas, aber auch um ein an der jeweiligen Komponente reiches Gemisch handeln.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Rektifikation zumindest teilweise aufzutrennen, also aus dem Stoffgemisch jeweils Reinstoffe oder zumindest Stoffgemische mit anderer Zusammensetzung zu erzeugen. Typischerweise sind Rektifikationskolonnen als zylindrische Metallbehälter ausgebildet, die mit Einbauten, beispielsweise Trennböden oder geordneten oder ungeordneten Packungen, ausgerüstet sind. Eine Rektifikationskolonne weist einen Sumpfverdampfer auf. Hierbei handelt es sich um eine Einrichtung mit einem Wärmetauscher, der beheizt wird, und der dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil des Sumpfprodukts verdampft und gasförmig in den Trennbereich zurückgespeist.

### Vorteile der Erfindung

Bei Verwendung von MoVNbTeOx-Katalysatoren für die ODH, insbesondere für die ODH-E, nimmt die Katalysatoraktivität über die Zeit ab, wenn ein dem jeweiligen Reaktor zugeführtes Gasgemisch kein oder nur sehr wenig Wasser beinhaltet. Dies führt bei Verwendung von nichtwässrigen Verdünnungsmitteln oder bei einem Reaktorbetrieb ganz ohne Verdünnungsmittel dazu, dass ein zeitlich konstanter Betrieb ohne stetige Nachregelung nicht möglich ist. In der Praxis bedeutet dies, dass die Temperatur im Reaktor stetig erhöht werden muss, um eine gleichbleibende Konversion beizubehalten.

Unter der "Konversion" bzw. dem "Umsatz" wird dabei hier der molare Anteil der eingesetzten Edukte, im Falle der ODH-E des Ethans, verstanden, der insgesamt zu (Haupt- und Neben-)Produkten reagiert. Die Einstellung der Temperatur in einem entsprechenden Reaktor erfolgt üblicherweise in Form einer Temperaturregelung des Kühlmediums, z.B. unter Verwendung eines Thermoöls oder einer Salzschmelze.

Wie bereits oben ausgeführt, entstehen bei entsprechenden Reaktionen neben dem gewünschten Zielprodukt (im Falle der ODH-E Ethylen) weitere sauerstoffhaltige Spezies wie die erwähnten Carbonsäuren, aber auch beispielsweise Carbonylverbindungen. Bei zu hohen Temperaturen wird ferner insbesondere die unerwünschte Bildung der Nebenprodukte Kohlenmonoxid und Kohlendioxid gefördert. Dies ist natürlich auch dann der Fall, wenn ein entsprechender Reaktor aufgrund der sich verringernden Katalysatoraktivität zur Aufrechterhaltung der Konversion bei zunehmend höherer Temperatur betrieben wird. Eine Temperaturerhöhung im Reaktor geht, mit anderen Worten, typischerweise mit einem Verlust an Selektivität und insbesondere der verstärkten Bildung von Kohlenmonoxid und Kohlendioxid einher. Dabei gilt es insbesondere zu beachten, dass kleine Änderungen der mittleren Reaktortemperatur lokal zu deutlich höheren Temperaturänderungen führen können und somit das System sehr empfindlich gegenüber Regeleingriffen reagieren kann.

Der Regelaufwand bei einer derartigen Regelung ist hoch. Zusätzlich werden sich durch die Anpassungen aber auch unvermeidbare Verschiebungen im Temperaturprofil über dem Katalysatorbett ergeben, die bei stark abnehmender Aktivität und hoher Kühlmitteltemperatur auch zu einem erhöhten Risiko eines thermischen Durchgangs führen. In letzter Konsequenz wird der Katalysatorbetrieb zeitlich stark begrenzt sein, d.h. dass bei Erreichen eines gewissen Schwellenwertes der Katalysator ersetzt oder, sofern technisch überhaupt möglich, in geeigneter Form regeneriert werden muss. Ferner weist eine Temperaturnachführung eine gewisse Trägheit auf, da insbesondere im Falle von Kühlmedien und Salzschmelzen das gesamte - in einem großtechnischen Prozess nicht unerhebliche - Inventar dieses Mediums entsprechend temperaturtechnisch nachgeführt werden muss.

Die vorliegende Erfindung beruht auf der Feststellung, dass die Katalysatoraktivität durch eine Wasserzugabe in den Reaktoreinsatzstrom geregelt werden kann. Hierzu wird eine die Katalysatoraktivität beschreibende Kenngröße, insbesondere mehrfach und unter konstanten voreingestellten Reaktionsbedingungen bzw. Prozesseinstellungen, ermittelt und die Wasserzugabe auf Grundlage dieser Kenngröße eingestellt. Insbesondere wird dabei auf Grundlage der mehrfach ermittelten wenigstens einen Kenngröße eine, insbesondere zeitliche, Zunahme oder Abnahme der Katalysatoraktivität ermittelt. Dies kann beispielsweise durch Ermitteln eines Trends aus nacheinander über einen bestimmten Zeitraum ermittelter Kenngrößen erfolgen. Liegt dabei eine Änderung vor, die betragsmäßig oberhalb eines definierten Schwellwerts liegt, kann durch Gegensteuern in Form der Wasserzugabe oder Wasserwegnahme hierauf reagiert werden. Die vorliegende Erfindung ermöglicht damit auch, entsprechende, ggf. kritische, Zustände vorab zu erkennen und kann dadurch beispielsweise ein thermisches Durchgehen sicher verhindern.

Auf diese Weise kann einerseits bei zu hoher Katalysatoraktivität und damit zu hohen Temperaturen im Reaktor ein thermisches Durchgehen des Reaktors unterbunden werden, indem die Wasserzugabe verringert oder unterbunden wird. Dies kann im Vergleich zu der trägen Anpassung des Kühlmediums sehr rasch erfolgen. Damit stellt die Verringerung der Wasserzugabe ein besonders vorteilhaftes Regulativ in einem entsprechenden Prozess dar. Diese kann auch beispielsweise als erster Schritt eines mehrstufigen Regelungsverfahrens erfolgen, in dem in einem zweiten Schritt die Temperatur des Kühlmediums mit entsprechend langsamerer Reaktion angepasst werden kann.

Andererseits kann im Rahmen der vorliegenden Erfindung auch dann, wenn eine zu geringe Katalysatoraktivität festgestellt wird, was beispielsweise dadurch erkannt werden kann, dass die Konversion abnimmt, die Aktivität durch eine entsprechende Wasserzugabe wieder erhöht werden. Auf diese Weise können die erwähnten Nachteile zu hoher Temperaturen und/oder zu steiler Temperaturgradienten, wie sie in den zuvor erläuterten herkömmlichen Verfahren auftreten, vermieden werden. Insbesondere kann im Rahmen der vorliegenden Erfindung die Bildung unerwünschter Nebenprodukte wie Kohlenmonoxid und Kohlendioxid im zeitlichen Verlauf und Mittel des Reaktorbetriebes insgesamt deutlich verringert werden. Die vorliegende Erfindung schafft also ein Verfahren mit insgesamt höherer Selektivität.

Im Rahmen der vorliegenden Erfindung wird ferner der hohe Regelaufwand einer derartigen herkömmlichen Regelung verringert bzw. vermieden. Im Rahmen der vorliegenden Erfindung können die erläuterten Verschiebungen im Temperaturprofil über dem Katalysatorbett verringert werden. Das Risiko eines thermischen Durchgangs wird auf diese Weise ebenfalls verringert. Die Verwendungszeit eines Katalysators kann im Rahmen der vorliegenden Erfindung erhöht werden.

Insgesamt kann im Rahmen der vorliegenden Erfindung durch die Einstellung der Wasserzugabe in den Reaktoreinsatzstrom eine gleichbleibende Katalysatoraktivität im jeweils gewünschten Bereich aufrechterhalten werden, wodurch ein entsprechendes Verfahren im Gegensatz zu bekannten Verfahren deutlich besser beherrschbar wird. Mit der vorliegenden Erfindung wird durch definierte Zugabe von Wasser eine konstante Katalysatoraktivität beibehalten, die zu einem deutlich reduzierten Regelaufwand, einer verbesserten Katalysatorlaufzeit und einem reduzierten Risiko eines thermischen Durchgangs führt. Dies kann sowohl bei Volllast- als auch bei Teillastbetrieb realisiert werden (dauerhafte Betriebspunkte). Gleichzeitig werden notwendige Temperaturanpassungen und -änderungen minimiert.

Die vorliegende Erfindung bietet ferner den Vorteil einer möglich werdenden, gestuften Reaktorabschaltung. Mittels einer ersten Deeskalationsstufe kann die Aktivität des Katalysators durch Wasserwegnahme schlagartig herabgesetzt werden, ohne aber die Reaktion komplett zu stoppen. Dadurch wird ein möglicher thermischer Durchgang zumindest herausgezögert, wenn nicht gar komplett abgefangen, bei gleichzeitiger Limitierung der Strömungsschwankungen im anschließenden Trennteil. Daraus folgen ein Zuwachs an Sicherheit und Anlagenverfügbarkeit (reduziertes Risiko für einen thermischen Durchgang), ein vereinfachter Betrieb (verlängerte Reaktionszeit für Betriebspersonal) und eine erhöhte Lebensdauer der Apparate im Trennteil (weniger Lastschwankungen). Ferner ergibt sich eine bessere Ausnutzung des Katalysators, da dieser durch stabileren Anlagenbetrieb zum einen eine längere Lebensdauer erreichen wird und zum anderen aufgrund der leichteren Nachregelung in einem größeren Betriebsfenster - also wiederum länger - betrieben werden kann.

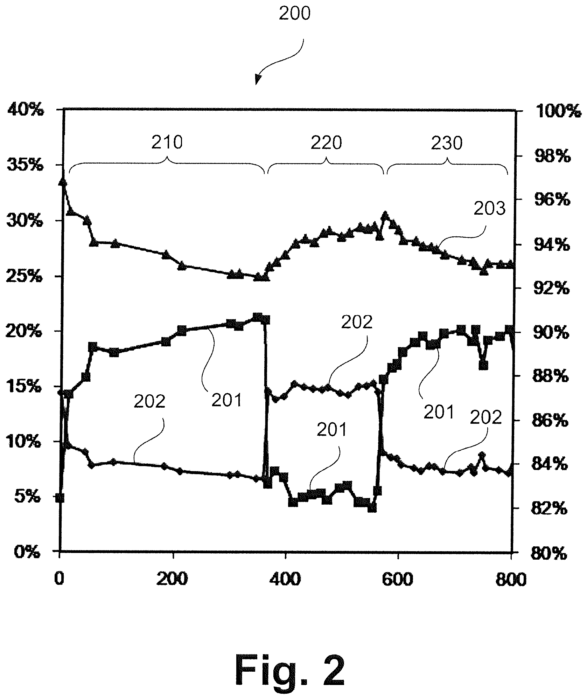

Ein entsprechender Reaktor wird insbesondere in einem festgelegten Betriebsbereich betrieben. Die Festlegung des hier vorgeschlagenen und unten näher erläuterten Betriebsbereiches resultiert maßgeblich aus detaillierten reaktionskinetischen Messungen an einem MoVNbTeOx-Katalysator in einem Laborreaktor. Bei diesen Ethanoxidationsversuchen wurde der Wassergehalt im Reaktionseinsatzstrom nach einer ersten trockenen Betriebszeit zunächst schlagartig von 0 auf 22 Vol.-% erhöht und später wieder schlagartig auf 0 Vol.-% reduziert, wie auch in Figur 2 veranschaulicht. Es zeigte sich, dass nach Hinzugabe von Wasser die Katalysatoraktivität (hier in Form der Ethankonversion) schnell zunimmt und im weiteren Verlauf weiter leicht ansteigt. Bei Wegnahme des Wassers beobachtet man erneut einen schnellen, kurzfristigen Abfall der Aktivität und darauffolgend weiter eine kontinuierliche Abnahme. Bei diesen Änderungen des Wassergehalts tritt zeitgleich eine sprungartige Änderung der Selektivitäten auf. Bei Zugabe von Wasser verschiebt sich die Produktselektivität nahezu instantan zu mehr Essigsäure, bei Wegnahme des Wassers zu mehr Ethylen.

Für das Teillastverhalten eines solchen Reaktors gilt somit, dass auch bei Teillast eine ausreichende (Mindest-)menge an Wasser im Feed beibehalten wird. In der Praxis ist es nämlich vorstellbar, dass bei Teillast (aufgrund in verringertem Umfang verfügbarer Edukte oder reduziertem Produktbedarf) vor allem die Konversion reduziert wird. Dies würde gemäß dem bisherigen Stand der Technik zunächst vor allem mittels Absenkung der Reaktortemperatur erfolgen, wodurch die Exothermie der Reaktion abnimmt und die Hinzugabe eines Verdünnungsmittels unter Umständen nicht mehr zwingend notwendig ist. Die Ausführung der vorliegenden Erfindung wird jedoch insbesondere gerade dadurch charakterisiert, dass auch in diesem Fall der angegebene Mindestanteil an Wasser im Feed beibehalten wird und so auch bei Teillast ein dauerhafter Betriebsfall vorliegt für den ansonsten eine Abnahme der Katalysatoraktivität schädlich wäre.

Im Umkehrschluss kann der beobachtete physikalische Effekt im Rahmen dieser Erfindung auch für eine gestufte Reaktorabschaltung bei Auftreten eines "Temperatur Hoch"-Alarms verwendet werden. Dieses Ereignis stellt einen nicht dauerhaften Betrieb dar, bei dem die Wasserzufuhr unter die angegebenen Grenzwerte und im Extremfall bis auf null gesenkt wird. In einer solchen gestuften Reaktorabschaltung kann in einem ersten Schritt die Wasserzufuhr zum Reaktor signifikant reduziert oder komplett gestoppt werden, um eine Abnahme der Katalysatoraktivität zu erreichen. Dabei erfolgt eine sehr schnelle Reduktion der vergleichsweise exothermen Bildung von Essigsäure und der entsprechenden Wärmefreisetzung im Reaktor.

Dabei wird der Umstand ausgenutzt, dass die Bildung von Essigsäure deutlich exothermer als die Reaktion von Ethan zu Ethylen und Wasser ist. Die entsprechenden Reaktionsenthalpien sind zu den nachfolgenden Gleichungen (1) und (2) angegeben:

C₂H₆ + 0,5 O₂ → C₂H₄ + H₂O, ΔH⁰_{R} = -105 kJ/mol (1)

C₂H₆ + 1,5 O₂ → CH₃COOH + H₂O, ΔH⁰_{R} = - 642 kJ/mol (2)

Gleichzeitig kann Stickstoff oder ein anderes geeignetes Verdünnungsmedium die entfallene Wassermenge teilweise oder ganz ersetzen. Gleichzeitig kann auch die dosierte Sauerstoffmenge reduziert werden, um den Sauerstoffanteil im Prozessgas am Reaktoraustritt konstant zu halten. Damit wird die Reaktion zwar weiterlaufen, jedoch deutlich gemäßigter, so dass eine Stabilisierung der Reaktortemperaturen auf einem bestimmten Niveau erreicht werden kann ohne nennenswerten Eingriff in die Temperatur eines Kühlmediums. Eine weitreichendere Abschaltung des Reaktors (z.B. Herunterfahren des eingespeisten Reaktionseinsatzstromes) kann als zusätzliche Deeskalationsstufe vorgesehen werden.

Die vorliegende Erfindung schlägt ein Verfahren zur Herstellung eines Olefins, insbesondere von Ethylen, vor, bei dem ein Reaktionseinsatzstrom gebildet wird, der zumindest ein Paraffin, insbesondere Ethan, Sauerstoff und Wasser enthält, und bei dem ein Teil des Paraffins und des Sauerstoffs in dem Reaktionseinsatzstrom unter Erhalt eines Prozessgases durch ODH, insbesondere durch ODH-E, unter Verwendung eines Katalysators zu dem Olefin umgesetzt wird, wobei das Prozessgas zumindest den nicht umgesetzten Teil des Paraffins und des Sauerstoffs, das Olefin und das Wasser aus dem Reaktionseinsatzstrom enthält. Wie erwähnt, kann das Prozessgas auch eine Reihe weiterer Komponenten enthalten, insbesondere Nebenprodukte wie organische Säuren (im Falle der ODH-E insbesondere Essigsäure), Kohlenmonoxid und Kohlendioxid. Weitere Komponenten sind beispielsweise sich in der ODH inert verhaltende Gase, darunter klassische Inertgase wie Stickstoff und/oder Edelgase, aber auch sich inert verhaltende Gase wie Methan.

Ein "Reaktionseinsatzstrom" ist im hier verwendeten Sprachgebrauch das gesamte, der ODH unterworfene Gasgemisch. Dieses kann insbesondere auch in Form separater Stoffströme dem oder den verwendeten Reaktoren zugespeist werden. Beispielsweise können ein paraffinhaltiger Stoffstrom und ein sauerstoffhaltiger Stoffstrom zu einem entsprechenden Reaktionseinsatzstrom in dem oder den verwendeten Reaktoren oder stromauf des oder der Reaktoren vereinigt werden.

Ein "Bilden" des Reaktionseinsatzstromes kann jegliche verfahrenstechnische Behandlung wie Verdichtung, Entspannung, Abkühlung oder Erwärmung oder auch die Abtrennung von Teilströmen, die Zuspeisung weiterer Stoffströme oder eine chemische Umsetzung von Komponenten umfassen. Insbesondere umfasst im Rahmen der vorliegenden Erfindung das Bilden des Reaktionseinsatzstromes beispielsweise, einen in den Reaktionseinsatzstrom überführten Stoffstrom zu erwärmen. Bei dieser Erwärmung, der sogenannten Feedvorwärmung, kann der Reaktionseinsatzstrom auf eine Temperatur gebracht werden, die die ODH in einer sich anschließenden Reaktionseinheit mit einem oder mehreren Reaktoren anlaufen lässt.

Erfindungsgemäß ist vorgesehen, dass zumindest eine Kenngröße ermittelt wird, welche eine Aktivität des Katalysators anzeigt, und dass eine Menge des Wassers in dem Reaktionseinsatzstrom auf Grundlage der zumindest einen ermittelten Kenngröße eingestellt wird. Die wenigstens eine Kenngröße wird dabei insbesondere mehrfach und insbesondere unter konstanten Prozesseinstellungen ermittelt. Die wenigstens eine Kenngröße kann beispielsweise kontinuierlich überwacht oder periodisch, beispielsweise zeitlich getaktet, ermittelt werden und dann, wenn eine Abweichung, insbesondere über einen vorgegebenen Schwellwert hinaus, vorliegt, oder ein Trend zurückliegender Messwerte in eine entsprechende Richtung weist, kann eine Einstellung der Wassermenge vorgenommen werden.

Die konstanten Prozesseinstellungen können insbesondere konstante Reaktionseinsatzparameter umfassen, wobei unter "konstanten Reaktionseinsatzparametern" insbesondere eine konstante Zusammensetzung, ein konstanter Druck, eine konstante Temperatur und/oder eine konstante Einsatzmenge des Reaktionseinsatzes, die dem oder den verwendeten Reaktoren bzw. der ODH pro Zeiteinheit zugeführt wird, und die zu einer entsprechend konstanten, auf die Katalysatormenge bezogenen Raumgeschwindigkeit führt, verstanden wird.

Konstante Prozesseinstellungen können insbesondere auch eine konstante Wärmeabfuhr, d.h. eine konstant eingestellte Leistung eines oder mehreren der verwendeten Reaktoren zugeordneten Kühlsystems, umfassen. Wird ein entsprechendes Kühlsystem konstant eingestellt, kann eine erhöhte Katalysatoraktivität insbesondere durch einen Temperaturanstieg im Reaktor erkannt werden.

Es versteht sich, dass "konstante" Prozesseinstellungen auch innerhalb eines gewissen Toleranzbereichs liegen können. Dies ist in der Regel der Fall, weil eine völlig exakte Einstellung, beispielsweise einer Zusammensetzung des Einsatzstroms oder von dessen Druck oder Temperatur, typischerweise aufgrund technischer Gegebenheiten wie Toleranzen in Dosiereinheiten oder Druck- und Temperaturgebern, nicht möglich ist. Konstante Prozesseinstellungen umfassen jedoch keine gezielte Änderung, beispielsweise der Zusammensetzung des Reaktionseinsatzes, sondern stellen in der Regel konstante Sollwertvorgaben für entsprechende Stellgrößen dar. Die Änderungen, die sich bei einem derartigen Vorgehen daher im Laufe der Zeit ergeben und über den oder die wenigstens eine Kenngröße feststellen lassen, sind daher nicht durch den Reaktionseinsatz bzw. dessen Parameter bzw. andere Prozesseinstellungen bedingt, sondern hängen insbesondere von der Katalysatoraktivität ab.

Wie bereits erwähnt, geben die US 2010/0256432 A1 und die WO 2013/148006 A1 an, dass Wasserdampf in der ODH als Wärmemoderator oder Verdünnungsmittel eingesetzt werden kann. Mit anderen Worten lehren diese Druckschriften, dass eine übermäßige Wärmeentwicklung im Reaktor dadurch verhindert werden kann, dass eine größere Menge an Wasserdampf im Reaktionseinsatz in den oder die verwendeten Reaktoren zugegeben wird. So wird hier insbesondere der Wasseranteil im Reaktionseinsatz für Betriebspunkte mit hoher Konversion erhöht. In diesen Fällen ermöglicht die vermehrte Zudosierung von Wasser, die vorab vorgegeben wird, einen übermäßigen Anstieg der Temperaturen und der Konversion im Reaktor zu vermeiden, wenn z.B. die mittlere Temperatur im Katalysatorbett durch eine Veränderung der Kühlsystemeinstellungen erhöht wird.

Die vorliegende Erfindung beruht nun aber auf genau der gegenteiligen Feststellung. So konnte im Rahmen der vorliegenden Erfindung ein gegenteiliger Einfluss des Wassergehalts auf die Temperaturen und die Konversion im Reaktor festgestellt werden. Die beispielsweise in der beigefügten Figur 2 gezeigten Zeitverlaufsdaten zeigen, wie die vermehrte Zudosierung von Wasser die Ethankonversion erhöht und die Selektivität zu Essigsäure in etwa dem Maße erhöht, wie es die Selektivität zu Ethylen reduziert. Nachdem die Reaktion des Ethans zur Essigsäure exothermer ist als die von Ethan zum Ethylen, handelt es sich hierbei nicht um eine Moderation der Reaktion durch Wasserhinzunahme, sondern um das genaue Gegenteil. Das Wasser verhält sich somit nicht wie ein konventionelles Verdünnungsmedium, sondern wirkt reaktionsfördernd. Dieses Katalysatorverhalten gilt vor allem in dem im Rahmen der vorliegenden Anmeldung als besonders bevorzugt erwähnten Bereich für den Wassergehalt. Ein wesentlicher Aspekt der vorliegenden Erfindung besteht somit im Ausnutzen dieses Umstandes für die beanspruchte Steuerung der Katalysatoraktivität, die sonst nur sehr schwer beeinflussbar ist.

Wie zuvor ausführlich hergeleitet, kann durch eine Einstellung des Wassergehalts in dem Reaktionseinsatzstrom die Katalysatoraktivität gesteuert werden. Auf die obigen Ausführungen wird daher verwiesen. Auch geeignete, die Aktivität des Katalysators anzeigende Größen, die im Rahmen der vorliegenden Erfindung zum Einsatz kommen können, wurden bereits zuvor erläutert. Auch nachfolgend wird nochmals auf solche Größen Bezug genommen.

Im Rahmen des erfindungsgemäßen Verfahrens kann insbesondere vorgesehen sein, dass die Menge des Wassers in dem Reaktionseinsatzstrom verringert wird, wenn die zumindest eine Kenngröße eine Aktivität des Katalysators anzeigt, die oberhalb eines vorgegebenen Werts liegt. Ein entsprechender Wert kann insbesondere ein geeigneter Schwellwert sein, der beispielsweise in einem geeigneten Abstand zu einem Wert definiert wird, bei dem ein thermisches Durchgehen eines entsprechenden Reaktors zu befürchten ist. Insbesondere kann dabei auf Grundlage einer mehrfachen Ermittlung der zumindest einen Kenngröße eine zeitliche Veränderung, beispielsweise in Form eines Trends, erkannt und mit einem entsprechenden Schwellwert verglichen werden. Bei Überschreiten des Schwellwerts kann auch eine gestufte Reaktion durchgeführt werden, bei dem zunächst die Wassermenge nur in einem gewissen Umfang und später stärker verringert wird, beispielsweise um zu steile Gradienten zu verhindern. Auch die Verwendung mehrerer entsprechender Schwellwerte, von Hysteresefunktionen, zeitlichen Abstufungen und dergleichen, ist möglich.

In Fortführung eines entsprechenden Regelschritts zur Vermeidung eines thermischen Durchgehens kann in dem erfindungsgemäßen Verfahren die oxidative Dehydrierung unter Verwendung des Katalysators in einem mit einem Kühlmedium gekühlten Reaktor durchgeführt werden, wobei eine Temperatur des Kühlmediums verringert wird, nachdem die Menge des Wassers in dem Reaktionseinsatzstrom verringert wurde. Wie bereits erwähnt, kann beispielsweise zur Verhinderung eines thermischen Durchgehens die Verringerung der Wassermenge einen ersten Eingreifschritt mit schneller Reaktionszeit darstellen, dem anschließend die langsamer reagierenden konventionellen Schritte, insbesondere unter Verwendung eines Kühlmediums, folgen.

Wie erwähnt, kann eine entsprechende Deeskalation zur Vermeidung eines thermischen Durchgehens auch weitergeführt werden, insbesondere durch Reduzierung des Einsatzes. Eine Ausführungsform der Erfindung sieht daher vor, zumindest eine Menge des zumindest einen Paraffins in dem Reaktionseinsatzstrom zu verringern, nachdem die Menge des Wassers in dem Reaktionseinsatzstrom verringert wurde. Auf diese Weise kann die Exothermie durch Verringerung der umgesetzten Eduktmengen reduziert werden.

In weitgehend analoger Weise zur Verringerung der Menge des Wassers in dem Reaktionseinsatzstrom, wenn die zumindest eine Kenngröße eine Aktivität des Katalysators anzeigt, die oberhalb eines vorgegebenen Werts liegt, kann auch eine Erhöhung der Menge des Wassers in dem Reaktionseinsatzstrom erfolgen, wenn die zumindest eine Kenngröße eine Aktivität des Katalysators anzeigt, die unterhalb eines vorgegebenen Werts liegt. Auch hier kann ein entsprechender Wert insbesondere ein geeigneter Schwellwert sein, der beispielsweise in einem geeigneten Abstand zu einem Wert definiert wird, bei dem eine zu geringe Konversion eintritt. Insbesondere kann auch hier auf Grundlage einer mehrfachen Ermittlung der zumindest einen Kenngröße eine zeitliche Veränderung, beispielsweise in Form eines Trends, erkannt und mit einem entsprechenden Schwellwert verglichen werden. Beim Unterschreiten eines entsprechenden Schwellwerts kann auch hier eine gestufte Reaktion durchgeführt werden, bei dem zunächst die Wassermenge nur in einem gewissen Umfang und später stärker erhöht wird, beispielsweise um zu steile Gradienten zu verhindern. Auch die Verwendung mehrerer entsprechender Schwellwerte, von Hysteresefunktionen, zeitlichen Abstufungen und dergleichen, ist hier möglich.

Gemäß einer besonders bevorzugten Ausführungsform der vorliegenden Erfindung ist die wenigstens eine Kenngröße eine die Exothermie der oxidativen Dehydrierung unter Verwendung des Katalysators anzeigende Kenngröße. Beispielsweise kann eine Reaktortemperatur unter Beibehaltung einer konstanten Kühlleistung gemessen werden. Beispielsweise ist eine Messung innerhalb eines Reaktors oder in dem Prozessgas (direkt) stromab des Reaktors möglich. Steigt oder fällt eine entsprechende Temperatur über bzw. unter einen geeigneten Schwellwert, kann dann eine Reaktion wie zuvor erläutert eingeleitet werden. Die Vorteile einer derartigen Regelung im Gegensatz zu einer konventionellen Regelung über Kühlmedien wurde ebenfalls bereits ausführlich erläutert.

Gemäß einer anderen vorteilhaften Ausführungsform, die auch in Kombination mit der soeben erläuterten Ausführungsform eingesetzt werden kann, ist die wenigstens eine Kenngröße eine die Konversion des wenigstens einen Paraffins zu einem oder mehreren Reaktionsprodukten anzeigende Kenngröße. Wie bereits erwähnt und auch unter Bezugnahme auf Figur 2 erläutert, kann insbesondere die Konversion von Ethan in der ODH-E unter den entsprechenden, jeweils erläuterten Reaktionsbedingungen als ein Maß für die Katalysatoraktivität eingesetzt werden.

Im Rahmen des erfindungsgemäßen Verfahrens wird die Menge des Wassers in dem Reaktionseinsatzstrom insbesondere zwischen 5 und 95 Vol.-%, insbesondere 10 und 50 Vol.-%, insbesondere 14 und 35 Vol.-% eingestellt. Mit anderen Worten ist stets eine Mindestwassermenge vorgesehen, die, wie zuvor erläutert, dafür sorgt, dass die Aktivität des Katalysators erhalten bleibt und nicht aufgrund Wassermangels absinkt. Daher ist keine Kompensation durch eine Reaktortemperaturerhöhung wie sie zuvor für klassische Verfahren beschrieben wurde, erforderlich. Im Notfall, d.h. zu einer raschen Verringerung der Temperatur, kann die Wassermenge auch auf Null reduziert werden.

Wie erwähnt kommt die vorliegende Erfindung insbesondere dann zum Einsatz, wenn in der oxidativen Dehydrierung ein Katalysator, der zumindest die Elemente Molybdän, Vanadium, Niob und optional Tellur enthält, also ein sogenannter MoVTeNbO-Katalysator, verwendet wird, weil sich bei Verwendung eines derartigen Katalysators Ethylen und Essigsäure bilden und ein auf diese Weise gebildetes Reaktionssystem sich entsprechend den erwähnten Gesetzmäßigkeiten verhält.

Mit besonderem Vorteil wird die oxidative Dehydrierung im Rahmen der vorliegenden Erfindung in einem Temperaturbereich von 240 bis 500 °C in einem Reaktorbett des oder der verwendeten Reaktoren durchgeführt. Insbesondere kann der Temperaturbereich bei 260 und 400 °C, besonders bevorzugt bei 280 bis 350 °C liegen. Der Gesamtdruck am Reaktoreintritt des oder der Reaktoren liegt vorzugsweise zwischen 1 und 10 bar (abs.), insbesondere zwischen 2 und 9 bar (abs.), weiter insbesondere zwischen 3 und 8 bar (abs.). Die Raumgeschwindigkeit im Reaktorbett des oder der Reaktoren (WHSV) liegt im Bereich zwischen 0,1 und 10 kg Ethan/(h x kg Katalysator), bevorzugt zwischen 0,5 und 5 kg Ethan/(h x kg Katalysator), besonders bevorzugt zwischen 0,7 und 3 kg Ethan/(h x kg Katalysator).

Das erfindungsgemäße Verfahren kann insbesondere unter Verwendung eines oder mehrerer, dem Reaktionseinsatzstrom zugegebener und in das Prozessgas übergehender Verdünnungsmittel durchgeführt werden. Die Verwendung entsprechender Verdünnungsmittel, die insbesondere sicherstellen, dass bei der stark exothermen ODH ein stabiler und sicherer Reaktorbetrieb gewährleistet wird, ist grundsätzlich bekannt. Wie erwähnt, kann zur Einstellung des gewünschten Wassergehalts in dem genannten Bereich insbesondere eine Zugabe von Wasser bzw. Wasserdampf in den Reaktionseinsatzstrom erfolgen. Dieses Wasser bzw. dieser Wasserdampf wirkt zugleich als Verdünnungsmittel. Alternativ oder zusätzlich können aber ein oder mehrere weitere Verdünnungsmittel verwendet werden.

Insbesondere können im Rahmen der vorliegenden Erfindung ein oder mehrere Verdünnungsmittel zum Einsatz kommen, das oder die aus der Gruppe ausgewählt ist oder sind, die aus Wasser, Methan, Stickstoff und wenigstens einem weiteren Inertgas besteht. Auch Kohlendioxid kann als Verdünnungsmittel zum Einsatz kommen. Entsprechende Verdünnungsmittel nehmen an der Reaktion in dem oder den Reaktoren nicht oder allenfalls zu einem geringen Anteil teil und gehen daher zumindest zum überwiegenden Teil in das Prozessgas über.

Die vorliegende Erfindung erstreckt sich auch auf eine Anlage zur Herstellung eines Olefins, die dafür eingerichtet ist, einen Reaktionseinsatzstrom zu bilden, der zumindest ein Paraffin, Sauerstoff und Wasser enthält, und die ferner dafür eingerichtet ist, einen Teil des Paraffins und des Sauerstoffs in dem Reaktionseinsatzstrom unter Erhalt eines Prozessgases durch oxidative Dehydrierung unter Verwendung eines Katalysators zu dem Olefin umzusetzen, wobei das Prozessgas zumindest den nicht umgesetzten Teil des Paraffins und des Sauerstoffs, das Olefin und das Wasser aus dem Reaktionseinsatzstrom enthält. Die Anlage zeichnet sich durch Mittel aus, die dafür eingerichtet sind, zumindest eine Kenngröße zu ermitteln, welche eine Aktivität des Katalysators anzeigt, und durch eine Steuer- und/oder Regeleinheit, die dafür eingerichtet ist, eine Menge des Wassers in dem Reaktionseinsatzstrom auf Grundlage der zumindest einen ermittelten Kenngröße einzustellen

Zu Merkmalen und Vorteilen einer entsprechenden Anlage sei auf die obigen Erläuterungen zu den Merkmalen und Vorteilen des Verfahrens verwiesen. Insbesondere ist eine derartige Anlage zur Durchführung eines Verfahrens gemäß den oben erläuterten spezifischen Ausgestaltungen eingerichtet und weist hierzu geeignete Mittel auf. Auch diesbezüglich sei auf die obigen Ausführungen verwiesen.

Die Erfindung wird nachfolgend unter Bezugnahme auf die beigefügten Zeichnungen näher erläutert, die unter anderem bevorzugte Ausführungsformen der vorliegenden Erfindung veranschaulichen.

Kurze Beschreibung der Zeichnungen
Figur 1 veranschaulicht eine Anlage zur Herstellung von Ethylen und Essigsäure gemäß einer Ausführungsform der Erfindung.
Figur 2 veranschaulicht die Beeinflussung einer Katalysatoraktivität durch Wasser, die im Rahmen der vorliegenden Erfindung eingesetzt werden kann.

### Ausführliche Beschreibung der Zeichnungen

In den nachfolgenden Figuren sind einander funktionell oder baulich entsprechende Elemente mit identischen Bezugszeichen angegeben und werden der Übersichtlichkeit halber nicht wiederholt erläutert. Werden nachfolgend Anlagenteile beschrieben, gelten die Erläuterungen zu diesen sinngemäß auch für die mittels dieser Anlagenteile implementierten Verfahrensschritte und umgekehrt.

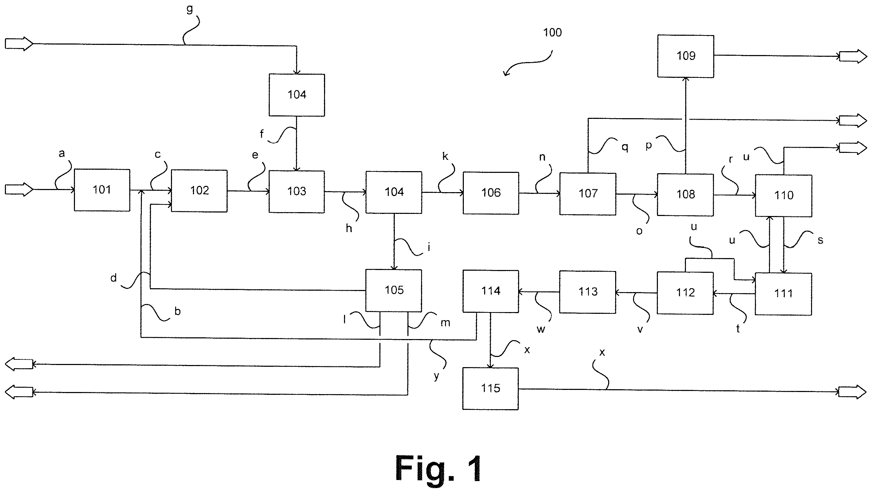

In Figur 1 ist eine Anlage zur Herstellung von Olefinen gemäß einer Ausführungsform der Erfindung in Form eines stark vereinfachten Anlagendiagramms veranschaulicht und insgesamt mit 100 bezeichnet. Wenngleich nachfolgend eine Anlage 100 zur ODH von Ethan (ODH-E) beschrieben wird, eignet sich, wie erwähnt, die vorliegende Erfindung auch zum Einsatz bei der ODH höherer Kohlenwasserstoffe. In diesem Fall gelten die nachfolgenden Erläuterungen entsprechend.

In der Anlage 100 wird einer Rektifikationseinheit 101 mit beispielsweise einer oder mehreren Rektifikationskolonnen ein Trenneinsatz in Form eines Stoffstroms a zugeführt und einer Rektifikation unterworfen. Der Trenneinsatz enthält im dargestellten Beispiel zumindest Ethan und höhere Kohlenwasserstoffe, insbesondere entsprechende höhere Paraffine. Der Rektifikationseinheit 101 können auch ein oder mehrere weitere Trenneinsätze zugeführt werden, beispielsweise der hier gezeigte und unten näher erläuterte Stoffstrom b.

In der Rektifikationseinheit 101 wird der Trenneinsatz alleine oder zusammen mit dem oder den weiteren Trenneinsätzen unter Erhalt eines Gasgemischs, das Ethan enthält, aber arm an höheren Kohlenwasserstoffen ist, einer Rektifikation unterworfen. Das Gasgemisch wird in Form eines Stoffstroms c abgezogen und einer Vorwärmeinheit 102 zugeführt. In der Vorwärmeinheit 102 wird das Gasgemisch vorgewärmt, wobei der Vorwärmeinheit 102 im dargestellten Beispiel auch ein Wasser- oder Dampfstrom d zugeführt wird. Auch weitere Stoffströme können zugeführt werden, wie hier in Form eines Stoffstroms b veranschaulicht. In der Rektifikationseinheit 101 wird ferner ein Komponentengemisch erhalten, das überwiegend oder ausschließlich die höheren Kohlenwasserstoffe enthält. Dieses ist nicht explizit gezeigt.

Ein aus der Vorwärmeinheit 102 abströmender Stoffstrom e wird zur Bildung eines Reaktionseinsatzstromes einer Reaktionseinheit 103 zugeführt. Der Reaktionseinsatzstrom enthält aufgrund seiner Bildung unter Verwendung des Trennprodukts aus der Rektifikationseinheit 101 Ethan, ist aber arm an höheren Kohlenwasserstoffen. Der Reaktionseinsatzstrom kann ferner ein oder mehrere Verdünnungsmittel wie Wasser oder Inertgase und weitere Komponenten enthalten. Diese können der Reaktionseinheit 103 auch in Form weiterer, nicht dargestellter Stoffströme zugeführt werden.

Der Reaktionseinheit 103 wird im dargestellten Beispiel ein sauerstoffhaltiger Stoffstrom f zugeführt. Dieser kann unter Verwendung einer Luftzerlegungsanlage 104 bereitgestellt werden. Der Luftzerlegungsanlage 104 wird hierzu ein Luftstrom g zugeführt. Bei dem sauerstoffhaltigen Stoffstrom f kann es sich um im Wesentlichen reinen Sauerstoff handeln, je nach Betrieb der Luftzerlegungsanlage 104 können jedoch auch Anteile an Stickstoff und an Edelgasen enthalten sein. Auf diese Weise ist es ebenfalls möglich, Verdünnungsmittel zuzuführen.

Aus der Reaktionseinheit 103 strömt ein Prozessgas in Form eines Prozessgasstroms h ab, in dem Ethylen enthalten ist, das in der Reaktionseinheit 103 durch ODH eines Teils des Ethans in dem Reaktionseinsatzstrom gebildet wurden. Ferner enthält das Produktgemisch Essigsäure, die ebenfalls bei der ODH in der Reaktionseinheit 103 aus Ethan gebildet wurde, Wasser, Kohlenmonoxid, Kohlendioxid, nicht umgesetzten Sauerstoff, sowie das oder die Verdünnungsmittel und weitere Verbindungen, falls zugegeben oder zuvor in der Reaktionseinheit 103 gebildet.

Es versteht sich, dass Reaktionseinheit 103 einen, aber auch mehrere, beispielsweise parallel betriebene, Reaktoren aufweisen kann. In letzterem Fall werden diesen Reaktoren jeweils entsprechende Reaktionseinsätze, die gleich oder unterschiedlich zusammengesetzt sein können, sowie entsprechende sauerstoffhaltige Stoffströme f zugeführt werden, und es werden jeweils entsprechende Prozessgasströme h gebildet. Letztere können beispielsweise vereinigt und gemeinsam als Prozessgas den nachfolgend erläuterten Einheiten zugeführt werden.

Das Prozessgas wird in eine Quencheinheit 104 überführt, in der es, beispielsweise in einer Waschsäule, mit Waschwasser oder einer geeigneten wässrigen Lösung in Kontakt gebracht werden kann. In der Quencheinheit 104 wird das Prozessgas insbesondere abgekühlt und die in der Reaktionseinheit 103 gebildete Essigsäure wird aus dem Prozessgas ausgewaschen. Mit Essigsäure beladenes Prozesswasser strömt in Form eines Stoffstroms i aus der Quencheinheit 104 ab, das zumindest weitgehend von Essigsäure befreite Prozessgas in Form eines Stoffstroms k.

In einer optionalen Essigsäurerückgewinnungseinheit 105 wird aus dem mit Essigsäure beladenen Prozesswasser Essigsäure als Eisessig abgetrennt, welcher als Stoffstrom I aus der Anlage 100 ausgeführt wird. In der Essigsäurerückgewinnungseinheit 105 ebenfalls rückgewonnenes reines Prozesswasser kann in Form des bereits erläuterten Stoffstroms d der Vorwärmeinheit 102 zugeführt werden. Das dem Reaktor zugeführte Prozesswasser kann auch teilweise oder ganz durch extern zugeführtes Frischwasser bereitgestellt werden. Nicht mehr brauchbares oder benötigtes Wasser kann in Form eines Abwasserstroms m aus der Anlage 100 ausgeführt und einer Abwasseraufbereitung zugeführt werden.

Das in Form des Stoffstroms k vorliegende, zumindest weitgehend von Essigsäure befreite Prozessgas wird in einer Verdichtungseinheit 106 auf ein geeignetes Druckniveau, beispielsweise 15 bis 25 bar, verdichtet und in Form eines verdichteten Stoffstroms n einer Aminwäscheeinheit 107 zugeführt. In dieser werden insbesondere Teile des in dem Prozessgas enthaltenen Kohlendioxids ausgewaschen. Nach Regenerierung des Amins kann das ausgewaschene Kohlendioxid in Form eines Stoffstroms q aus der Anlage ausgeführt werden. Das derart teilweise von Kohlendioxid befreite Prozessgas wird in Form eines Stoffstroms o in eine Laugenwäscheeinheit 108 überführt und dort weiter von Kohlendioxid gereinigt. In der Laugenwäscheeinheit 108 fällt Ablauge an, die in Form eines Stoffstroms p in eine Ablaugebehandlungseinheit 109 überführt und schließlich aus der Anlage 100 ausgeführt werden kann.

Das in der Laugenwäscheeinheit 108 weiter gereinigte Prozessgas wird in Form eines Stoffstroms r in eine Vorkühl- und Trockeneinheit 110 überführt, wo es insbesondere von Restwasser befreit werden kann. Das getrocknete Prozessgas wird in Form eines Stoffstroms s in eine Tieftemperatureinheit 111 überführt und anschließend in weiter abgekühlter Form in Form eines oder mehrerer Stoffströme t in eine Demethanisierungseinheit 112. In der Tieftemperatureinheit 111 und der Demethanisierungseinheit 112 werden tiefer als Ethylen siedende Komponenten, darunter insbesondere Kohlenmonoxid und Sauerstoff, aus dem Prozessgas abgetrennt, wobei der Rest in kondensierter Form verbleibt. Sind in dem Prozessgas höhere Kohlenwasserstoffe enthalten, die bei der ODH in der Reaktionseinheit 103 als Nebenprodukt gebildet wurden, gehen diese ebenfalls in das Kondensat über.

Die abgetrennten, tiefer als Ethylen siedenden Komponenten werden in Form eines oder mehrerer Stoffströme u durch die Tieftemperatureinheit 111 und die Vorkühl- und Trockeneinheit 110 zurückgeführt, dort ggf. mit weiteren entsprechenden Stoffströme vereinigt, zu Kühlzwecken genutzt, und aus der Anlage 100 ausgeführt. Die Kohlenwasserstoffe mit zwei und ggf. mehr Kohlenstoffatomen werden bei Bedarf in Form eines Stoffstroms v einer Hydriereinheit 113 zugeführt, in der insbesondere Acetylen, das ebenfalls bei der ODH in der in der Reaktionseinheit 103 als Nebenprodukt gebildet wurde, hydriert werden kann. Nach der Hydrierung wird der nun mit w bezeichnete Stoffstrom in eine Ethylenabtrenneinheit 114 überführt.

In der Ethylenabtrenneinheit 114 wird Ethylen zumindest weitgehend von anderen Komponenten abgetrennt und kann in Form eines Stoffstroms x nach Nutzung in einer Ethylenkälteeinheit 115 gasförmig aus der Anlage 100 ausgeführt werden. Die übrigen Komponenten, überwiegend Ethan und ggf. höhere Kohlenwasserstoffe, werden in Form eines Stoffstroms y abgezogen. Sind in diesem höhere Kohlenwasserstoffe enthalten, werden diese vorteilhafterweise in Form des bereits erwähnten Stoffstroms b in die Reaktionseinheit zurückgeführt. Es kann eine optionale Aufbereitung dieses Stoffstroms b erfolgen.

Durch einen angepassten Betrieb der Ethylenabtrenneinheit 114 bzw. eine entsprechende Auslegung kann ein Teil des Ethylens auch in den Stoffstrom y bzw. b überführt und in diesem in das Verfahren zurückgeführt werden. Es ist auch möglich, den Stoffstrom x oder einen Teil hiervon zurückzuführen. Auf diese Weise lässt sich, die Ausbeute an Essigsäure bei Bedarf erhöhen und damit die Selektivität anpassen. Es sei ausdrücklich betont, dass im Rahmen der vorliegenden Erfindung auf bestimmte Anlagenteile, insbesondere beispielsweise die Rektifikationseinheit 101, verzichtet werden kann. In diesem Fall kann der Stoffstrom y beispielsweise auch direkt in die Vorwärmeinheit 102 oder in die Reaktionseinheit 103 eingespeist werden. Weitere Varianten sind möglich und von der vorliegenden Erfindung umfasst.

Figur 2 veranschaulicht die Beeinflussung einer Katalysatoraktivität durch Wasser, die im Rahmen der vorliegenden Erfindung eingesetzt werden kann, anhand eines Diagramms 200. Das Diagramm 200 veranschaulicht Ergebnisse eines Versuchs, wie er zuvor bereits erläutert wurde und nachfolgend nochmals beschrieben wird.

In dem Diagramm 200 sind eine Selektivität zu Essigsäure und ein Umsatz von Ethan auf der linken Ordinate und eine Selektivität zu Ethylen auf der rechten Ordinate jeweils in Prozent gegenüber der Zeit in Minuten auf der Abszisse aufgetragen. Die Selektivitätsentwicklung zu Ethylen über die Zeit ist in Form eines Graphen 201, jene zu Essigsäure in Form eines Graphen 202 veranschaulicht. Die Entwicklung der Konversion von Ethan ist in Form eines Graphen 203 dargestellt.

Der Versuch erfolgte bei einer Raumgeschwindigkeit von 1,19 kg Ethan/(h x kg Katalysator) und einem festen molaren Verhältnis von Ethan zu Sauerstoff von 4 im Reaktoreinsatz. Für den Versuch wurden 48,8 g unverdünnter Katalysator in einen Laborreaktor mit 10 mm Innendurchmesser gefüllt.

Bei dem dargestellten Versuch wurde der Wassergehalt im Reaktionseinsatzstrom nach einem ersten "trockenen" Betriebszeitraum 210, während dessen kein Wasser in den Reaktionseinsatzstrom zugegeben wurde, zunächst schlagartig von 0 auf 22 Vol.-% erhöht, während eines Betriebszeitraums 220 auf diesem Wert beibehalten und danach wieder schlagartig auf 0 Vol.-% reduziert und während eines Betriebszeitraums 230 wiederum auf diesem Wert beibehalten.

Es zeigt sich, dass nach Hinzugabe von Wasser die Katalysatoraktivität (hier in Form der Ethankonversion, Graph 203) schnell zunimmt und im weiteren Verlauf des Betriebszeitraums 220 weiter leicht ansteigt. Bei Wegnahme des Wassers beobachtet man erneut einen schnellen, kurzfristigen Abfall der Aktivität und darauffolgend weiter eine kontinuierliche Abnahme über den Betriebszeitraum 230. Bei diesen Änderungen des Wassergehalts tritt zeitgleich eine sprungartige Änderung der Selektivitäten (zu Ethylen und Essigsäure, Graphen 201 und 202) auf. Bei Zugabe von Wasser verschiebt sich die Produktselektivität nahezu instantan zu mehr Essigsäure, bei Wegnahme des Wassers zu mehr Ethylen.

## Patentansprüche

1. Verfahren zur Herstellung eines Olefins, bei dem ein Reaktionseinsatzstrom gebildet wird, der zumindest ein Paraffin, Sauerstoff und Wasser enthält, und bei dem ein Teil des Paraffins und des Sauerstoffs in dem Reaktionseinsatzstrom unter Erhalt eines Prozessgases durch oxidative Dehydrierung unter Verwendung eines Katalysators zu dem Olefin umgesetzt wird, wobei das Prozessgas zumindest den nicht umgesetzten Teil des Paraffins und des Sauerstoffs, das Olefin und das Wasser aus dem Reaktionseinsatzstrom enthält,
**dadurch gekennzeichnet, dass** zumindest eine Kenngröße ermittelt wird, welche eine Aktivität des Katalysators anzeigt, und dass eine Menge des Wassers in dem Reaktionseinsatzstrom auf Grundlage der zumindest einen ermittelten Kenngröße eingestellt wird.

2. Verfahren nach Anspruch 1, bei dem die Menge des Wassers in dem Reaktionseinsatzstrom verringert wird, wenn die zumindest eine Kenngröße eine Aktivität des Katalysators anzeigt, die oberhalb eines vorgegebenen Werts liegt.

3. Verfahren nach Anspruch 2, bei dem die oxidative Dehydrierung unter Verwendung des Katalysators in einem mit einem Kühlmedium gekühlten Reaktor durchgeführt wird, wobei eine Temperatur des Kühlmediums verringert wird, nachdem die Menge des Wassers in dem Reaktionseinsatzstrom verringert wurde.

4. Verfahren nach Anspruch 2 oder 3, bei dem zumindest eine Menge des Sauerstoffs in dem Reaktionseinsatzstrom verringert wird, nachdem die Menge des Wassers in dem Reaktionseinsatzstrom verringert wurde.

5. Verfahren nach Anspruch 2, 3 oder 4, bei dem zumindest eine Menge des zumindest einen Paraffins in dem Reaktionseinsatzstrom verringert wird, nachdem die Menge des Wassers in dem Reaktionseinsatzstrom verringert wurde.

6. Verfahren nach einem der vorstehenden Ansprüche, bei dem die Menge des Wassers in dem Reaktionseinsatzstrom erhöht wird, wenn die zumindest eine Kenngröße eine Aktivität des Katalysators anzeigt, die unterhalb eines vorgegebenen Werts liegt, und/oder wenn die zumindest eine Kenngröße eine zeitliche Abnahme einer Aktivität des Katalysators anzeigt.

7. Verfahren nach einem der vorstehenden Ansprüche, bei dem die wenigstens eine Kenngröße eine die Exothermie der oxidativen Dehydrierung unter Verwendung des Katalysators anzeigende Kenngröße, eine die Konversion des wenigstens einen Paraffins zu einem oder mehreren Reaktionsprodukten anzeigende Kenngröße und/oder eine Temperatur in einem für die oxidative Dehydrierung eingesetzten Reaktor oder stromab dieses Reaktors ist.

8. Verfahren nach einem der vorstehenden Ansprüche, bei dem die Menge des Wassers in dem Reaktionseinsatzstrom zwischen 5 und 95 Vol.-%, insbesondere 10 und 50 Vol.-%, insbesondere 14 und 35 Vol.-% eingestellt wird.

9. Verfahren nach einem der vorstehenden Ansprüche, bei dem die oxidative Dehydrierung bei einer Temperatur des Katalysators in einem Bereich zwischen 240 und 500°C durchgeführt wird.

10. Verfahren nach einem der vorstehenden Ansprüche, bei dem für die oxidative Dehydrierung ein oder mehrere Reaktoren verwendet werden und bei dem der Gesamtdruck des Reaktionseinsatzstromes am Eintritt des oder der Reaktoren in einem Bereich zwischen 1 und 10 bar (abs.) liegt.

11. Verfahren nach einem der vorstehenden Ansprüche, bei dem die oxidative Dehydrierung bei einer Raumgeschwindigkeit in einem Bereich zwischen 0,1 und 10 kg Ethan/(h x kg Katalysator) durchgeführt wird.

12. Verfahren nach einem der vorstehenden Ansprüche, bei dem dem Reaktionseinsatzstrom ferner ein oder mehrere, in das Prozessgas übergehende Verdünnungsmittel zugegeben werden.

13. Verfahren nach Anspruch 12, bei dem das oder die Verdünnungsmittel aus der Gruppe ausgewählt ist oder sind, die aus Methan, Stickstoff, Kohlendioxid und wenigstens einem weiteren Inertgas besteht.

14. Verfahren nach einem der vorangegangenen Ansprüche, wobei der verwendete Katalysator zumindest die Elemente Molybdän, Vanadium, Niob und optional Tellur enthält.

## Claims

1. Method for manufacturing an olefin, wherein a reaction feed stream is formed which contains at least one paraffin, oxygen and water and wherein one portion of the paraffin and of the oxygen in the reaction feed stream is reacted to the olefin by oxidative dehydrogenation using a catalyst by yielding a process gas, wherein the process gas contains at least the unreacted portion of the paraffin and of the oxygen, the olefin and the water from the reaction feed stream,
**characterized in that** at least one characteristic is determined which indicates an activity of the catalyst and that an amount of the water in the reaction feed stream is adjusted based on the at least one determined characteristic.

2. Method according to claim 1, wherein the amount of water in the reaction feed stream is reduced if the at least one characteristic indicates an activity of the catalyst that is above a predetermined value.

3. Method according to claim 2, wherein the oxidative dehydrogenation is carried out using the catalyst in a reactor cooled with a cooling medium, wherein a temperature of the cooling medium is reduced after the amount of water in the reaction feed stream has been reduced.

4. Method according to claim 2 or 3, wherein at least an amount of the oxygen in the reaction feed stream is reduced after the amount of water in the reaction feed stream has been reduced.

5. Method according to claim 2, 3 or 4, wherein at least an amount of the at least one paraffin in the reaction feed stream is reduced after the amount of water in the reaction feed stream has been reduced.

6. Method according to any one of the preceding claims, wherein the amount of water in the reaction feed stream is increased if the at least one characteristic indicates an activity of the catalyst which is below a predetermined value and/or if the at least one characteristic indicates a temporal decrease in activity of the catalyst.

7. Method according to any one of the preceding claims, wherein the at least one characteristic is a characteristic indicating the exothermicity of the oxidative dehydrogenation using the catalyst, a characteristic indicating the conversion of the at least one paraffin to one or more reaction products, and/or a temperature in a reactor used for the oxidative dehydrogenation or downstream of said reactor.

8. Method according to any one of the preceding claims, wherein the amount of water in the reaction feed stream is set to between 5 and 95% by volume, in particular 10 and 50% by volume, in particular 14 and 35% by volume.

9. Method according to any one of the preceding claims, wherein the oxidative dehydrogenation is carried out at a temperature of the catalyst in a range between 240 and 500°C.

10. Method according to any one of the preceding claims, wherein one or more reactors are used for the oxidative dehydrogenation and wherein the total pressure of the reaction feed stream at the inlet of the reactor or reactors is in a range between 1 and 10 bar (abs.).

11. Method according to any one of the preceding claims, wherein the oxidative dehydrogenation is carried out at a space velocity in a range between 0.1 and 10 kg of ethane/(h x kg of catalyst).

12. Method according to any one of the preceding claims, wherein one or more diluents which pass into the process gas are furthermore added to the reaction feed stream.

13. Method according to claim 12, wherein the diluent or diluents are selected from the group consisting of methane, nitrogen, carbon dioxide, and at least one further inert gas.

14. Method according to any one of the preceding claims, wherein the catalyst used comprises at least the elements molybdenum, vanadium, niobium and optionally tellurium.

## Revendications

1. Procédé de préparation d'une oléfine, selon lequel un flux réactionnel de départ est formé, lequel contient au moins une paraffine, de l'oxygène et de l'eau, et selon lequel une partie de la paraffine et de l'oxygène dans le flux réactionnel de départ est transformée en oléfine par déshydrogénation oxydative à l'aide d'un catalyseur avec obtention d'un gaz de procédé, le gaz de procédé contenant au moins la partie n'ayant pas réagi de la paraffine et de l'oxygène, l'oléfine et l'eau provenant du flux réactionnel de départ, **caractérisé en ce qu'**au moins une grandeur caractéristique est déterminée, laquelle indique une activité du catalyseur, et **en ce qu'**une quantité d'eau dans le flux réactionnel de départ est réglée en fonction de ladite au moins une grandeur caractéristique déterminée.

2. Procédé selon la revendication 1, selon lequel la quantité d'eau dans le flux réactionnel de départ est réduite lorsque ladite au moins une grandeur caractéristique indique une activité du catalyseur, laquelle est supérieure à une valeur prédéterminée.

3. Procédé selon la revendication 2, selon lequel la déshydrogénation oxydative est effectuée à l'aide du catalyseur dans un réacteur refroidi au moyen d'un milieu de refroidissement, une température du milieu de refroidissement étant réduite après que la quantité d'eau dans le flux réactionnel de départ a été réduite.

4. Procédé selon la revendication 2 ou 3, selon lequel au moins une quantité d'oxygène dans le flux réactionnel de départ est réduite après que la quantité d'eau dans le flux réactionnel de départ a été réduite.

5. Procédé selon la revendication 2, 3 ou 4, selon lequel au moins une quantité de ladite au moins une paraffine dans le flux réactionnel de départ est réduite après que la quantité d'eau dans le flux réactionnel de départ a été réduite.

6. Procédé selon l'une quelconque des revendications précédentes, selon lequel la quantité d'eau dans le flux réactionnel de départ est augmentée lorsque ladite au moins une grandeur caractéristique indique une activité du catalyseur, laquelle est inférieure à une valeur prédéterminée et/ou lorsque ladite au moins une grandeur caractéristique indique une diminution temporelle d'une activité du catalyseur.

7. Procédé selon l'une quelconque des revendications précédentes, selon lequel ladite au moins une grandeur caractéristique est une grandeur caractéristique indiquant l'exothermicité de la déshydrogénation oxydative à l'aide du catalyseur, une grandeur caractéristique indiquant la conversion de ladite au moins une paraffine en un ou plusieurs produit(s) réactionnel(s) et/ou une température dans un réacteur employé pour la déshydrogénation oxydative ou en aval de ce réacteur.

8. Procédé selon l'une quelconque des revendications précédentes, selon lequel la quantité d'eau dans le flux réactionnel de départ est réglée entre 5 et 95 % en volume, en particulier entre 10 et 50 % en volume, en particulier entre 14 et 35 % en volume.

9. Procédé selon l'une quelconque des revendications précédentes, selon lequel la déshydrogénation oxydative est effectuée à une température du catalyseur dans une plage entre 240 et 500 °C.

10. Procédé selon l'une quelconque des revendications précédentes, selon lequel, pour la déshydrogénation oxydative, un ou plusieurs réacteur(s) est/sont utilisé(s) et selon lequel la pression totale du flux réactionnel de départ à l'entrée dudit un ou desdits plusieurs réacteur(s) est située dans la plage entre 1 et 10 bars (abs.).

11. Procédé selon l'une quelconque des revendications précédentes, selon lequel la déshydrogénation oxydative est effectuée à une vitesse spatiale dans la plage entre 0,1 et 10 kg d'éthane/(h x kg de catalyseur).

12. Procédé selon l'une quelconque des revendications précédentes, selon lequel un ou plusieurs diluant(s) passant dans le gaz de procédé est/sont en outre ajouté(s) au flux réactionnel de départ.

13. Procédé selon la revendication 12, selon lequel le ou les diluant(s) est/sont choisi(s) dans le groupe constitué par le méthane, l'azote, le dioxyde de carbone et au moins un autre gaz inerte.

14. Procédé selon l'une quelconque des revendications précédentes, selon lequel le catalyseur utilisé contient au moins les éléments molybdène, vanadium, niobium et éventuellement tellure.
